# FASCICULE DE BREVET EUROPEEN

(11) **EP 2 396 044 B1**
(45) Date de publication et mention de la délivrance du brevet: **09.01.2019**
(21) Numéro de dépôt: 10707095.5
(22) Date de dépôt: 12.02.2010
(51) Int. Cl.: A61L 15/26, A61F 13/06, A01F 13/00, A61K 8/72, A61Q 19/00

(54) **MATERIAU POUR LA PREVENTION DES ESCARRES**
MATERIAL ZUR VERHINDERUNG VON DEKUBITALGESCHWÜREN
MATERIAL FOR THE PREVENTION OF BEDSORES

(30) Priorité: 13.02.2009 FR 0900664
(43) Date de publication de la demande: 21.12.2011
(73) Titulaire: Millet Innovation, 26270 Loriol sur Drome (FR)
(72) Inventeur: MILLET, Damien, F-26000 Valence (FR); JOURDAN, Eric, F-38340 Voreppe (FR)
(74) Mandataire: de Roquemaurel, Bruno
(86) Numéro de dépôt international: PCT/FR2010/000116
(87) Numéro de publication internationale: WO 2010/092259

(56) Documents cités:
- WO-A1-02/17840
- FR-A1- 2 712 487
- DATABASE WPI Week 200675 Thomson Scientific, London, GB; AN 2006-717956 XP002556614 & CN 1 748 758 A (FUKANGREN BIOLOGICAL PHARM SCI & TECH CO) 22 mars 2006 (2006-03-22)

## Description

La présente invention concerne un matériau notamment pour la prévention des escarres.

Divers moyens tels que crèmes, coussins et matériaux doux ont été employés pour tenter de prévenir la formation d'escarres. Cependant, aucun de ces moyens ne se révèle vraiment efficace.

Il est également connu d'utiliser des plaquettes à base de gel polymère tel que du gel de silicone ou à base d'hydrogel pour assurer une fonction de protection de la peau ou de répartition de charge. Par exemple pour assurer une fonction de répartition de charge, il est connu d'utiliser une plaquette en un gel de silicone de type PDMS (polydiméthylsiloxane) relativement dure. Le brevet FR2712487 décrit un gel de silicone ayant des propriétés se rapprochant de celles du capiton plantaire pour la prévention de pathologies d'hyper appuis apparaissant essentiellement sur ou sous les pieds.

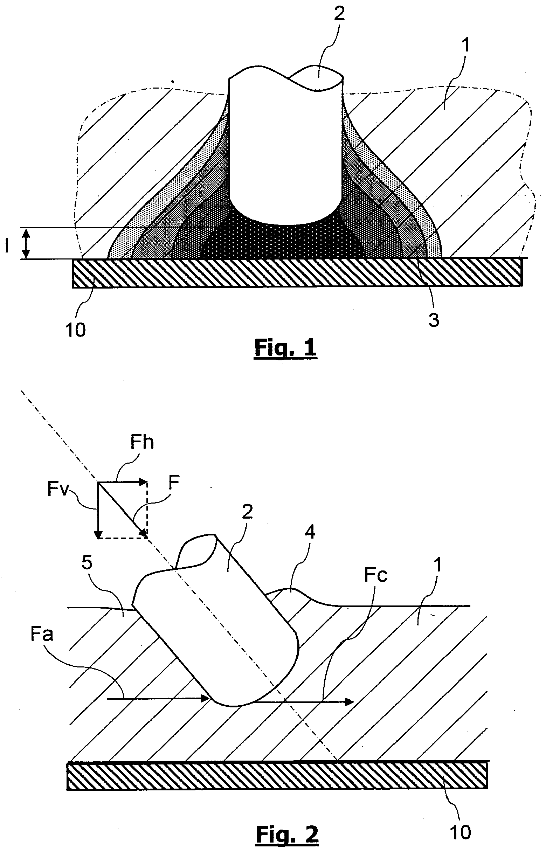

Toutefois, la formation d'escarre résulte de forces s'exerçant sur les tissus qui diffèrent des hyper appuis. Les figures 1 et 2 représentent schématiquement en coupe une zone de peau et de tissus 1 autour d'un os 2 en appui sur une surface rigide 10. Sur la figure 1, l'os 2 se trouve séparé de la surface rigide 10 par une couche de tissus d'épaisseur minimum 1. La pression exercée sur la surface rigide conduit à la formation d'un champ de forces représenté en zones grisées 3, dont le niveau de gris est représentatif de l'intensité des forces. La zone la plus foncée située entre l'os 2 et la surface rigide délimite la zone ou les forces d'appui sont les plus intenses.
Les tissus se trouvent ainsi le plus comprimés dans l'axe de l'os 2, et de moins en moins comprimés en s'éloignant de l'os.

En réalité, l'appui d'un os n'est pas nécessairement perpendiculaire à la surface d'appui. Les phénomènes qui se produisent dans cette situation sont illustrés par la figure 2. La force F exercée par l'os comporte une composante verticale Fv et une composante horizontale Fh. La composante verticale peut créer un hyper appui et former une zone 5 de rétrécissement de l'épaisseur de la peau d'un côté de l'os. Le rétrécissement induit une mauvaise irrigation des tissus dans cette zone. La composante horizontale Fh induit une force de cisaillement Fa et une force de compression Fc dans les tissus de part et d'autre de l'os 2, qui ont tendance à fatiguer les tissus notamment en raison de mouvements de frottement sur la surface d'appui 10. Les vaisseaux subissent également ces contraintes. La peau et les tissus plus en profondeur sont donc mal irrigués en raison de la compression, et subissent des contraintes alternatives parallèles à la surface d'appui qui ont d'autant plus d'effets néfastes que les tissus sont moins élastiques comme c'est le cas chez les personnes âgées ou mal nutries. A la surface de la peau, ces mouvements de frottement peuvent également conduire à la formation de lésions. Les forces de compression, cisaillement et frottement ont donc tendance à former une escarre.

Il est donc souhaitable de mettre au point un matériau à interposer entre la peau et la surface d'appui qui permette de supprimer, ou à défaut d'amoindrir les effets néfastes d'appuis et de frottements sur la peau.

La demande de brevet WO 02/17840 décrit un pansement destiné au traitement notamment d'escarres. Le pansement est constitué d'un matériau textile souple extensible, maintenant en place sur la zone du corps blessée un tampon en un gel polymère de silicone moulé et présentant une dureté Shore A de 6 à 8. Il s'avère que ce tampon ne permet pas de prévenir la formation d'escarres.

Des modes de réalisation peuvent concerner un procédé de fabrication d'une couche de protection contre les escarres, comprenant des étapes de formation d'un mélange, de polymérisation au moins partielle du mélange pour obtenir un gel polymère, et de formation d'une couche de protection à partir du gel polymère, le mélange comprenant :
15% de polydiméthylsiloxane à terminaisons diméthyl-vinyles ayant une viscosité supérieure à 20000 mPa.s,
25% de polydiméthylsiloxane à terminaisons diméthyl-vinyles ayant une viscosité comprise entre 200 et 20000 mPa.s,
45% de polydiméthylsiloxane à terminaisons triméthyles,
12% de silice de pyrogénation traitée triméthylsiloxy, et
3% de co-polydiméthylsiloxane-polyméthyl-hydrogéno-siloxane à terminaisons diméthyle-hydrogéno, le gel polymère présentant à 35°C, lorsqu'une fréquence de sollicitation varie entre 0 et 100 rd/s, une rigidité ou composante élastique variant de 11000 à 20000 Pa, une viscosité ou composante amortissante variant de 700 à 8000 Pa et un facteur d'amortissement ou Tan Delta variant de 0,06 à 0,38.

Selon un mode de réalisation, la couche de protection présente une épaisseur comprise entre 1 et 4 mm.

Selon un mode de réalisation, une face de la couche de protection présente un état de surface obtenu par une polymérisation libre de toute pression contre une surface lisse.

Selon un mode de réalisation, les 45% de polydiméthylsiloxane à terminaisons triméthyles présentent une viscosité inférieure à 100 mPa.s.

Selon un mode de réalisation, les 45% de polydiméthylsiloxane à terminaisons triméthyles comprennent environ 5/9ième de polydiméthylsiloxane ayant une viscosité inférieure à 100 mPa.s, et environ 4/9ième de polydiméthylsiloxane ayant une viscosité supérieure à 20000 mPa.s.

Selon un mode de réalisation, la polymérisation est réalisée en présence de platine complexé vinylesiloxanes.

Des modes de réalisation peuvent également concerner une couche de protection contre les escarres, comprenant un gel polymère obtenu par polymérisation au moins partielle d'un mélange comprenant :
15% de polydiméthylsiloxane à terminaisons diméthyl-vinyles ayant une viscosité supérieure à 20000 mPa.s,
25% de polydiméthylsiloxane à terminaisons diméthyl-vinyles ayant une viscosité comprise entre 200 et 20000 mPa.s,
45% de polydiméthylsiloxane à terminaisons triméthyles,
12% de silice de pyrogénation traitée triméthylsiloxy, et
3% de co-polydiméthylsiloxane-polyméthyl-hydrogéno-siloxane à terminaisons diméthyle-hydrogéno, le gel polymère présentant à 35°C, lorsqu'une fréquence de sollicitation varie entre 0 et 100 rd/s, une rigidité ou composante élastique variant de 11000 à 20000 Pa, une viscosité ou composante amortissante variant de 700 à 8000 Pa et un facteur d'amortissement ou Tan Delta variant de 0,06 à 0,38.

Selon un mode de réalisation, la couche de protection présente une épaisseur comprise entre 2 et 3 mm.

Selon un mode de réalisation, la couche de protection comprend une face présentant un état de surface obtenu par une polymérisation libre de toute pression contre une surface lisse.

Selon un mode de réalisation, les environ 45% de polydiméthylsiloxane à terminaisons triméthyles présentent une viscosité inférieure à 100 mPa.s.

Selon un mode de réalisation, les environ 45% de polydiméthylsiloxane à terminaisons triméthyles comprennent environ 5/9ième de polydiméthylsiloxane ayant une viscosité inférieure à 100 mPa.s, et environ 4/9ième de polydiméthylsiloxane ayant une viscosité supérieure à 20000 mPa.s.

Selon un mode de réalisation, la polymérisation est réalisée en présence de platine complexé vinylesiloxanes.

Des modes de réalisation peuvent également concerner un produit pour restaurer ou renforcer le réflexe de vasodilatation des capillaires sanguins cutanés, induit par l'application d'une pression locale, comprenant une couche de protection telle que précédemment définie.

Des exemples de réalisation de l'invention seront décrits dans ce qui suit, à titre non limitatif en relation avec les figures jointes parmi lesquelles :
les figures 1 et 2 décrites précédemment représentent schématiquement en coupe une zone de peau et de tissus autour d'un os,
la figure 3 représente schématiquement en coupe une zone de peau et de tissus autour d'un os, recouverte d'une couche de protection réalisée dans un matériau selon un mode de réalisation,
les figures 4 à 6 sont des courbes de variations de propriétés d'une couche de protection selon un mode de réalisation.

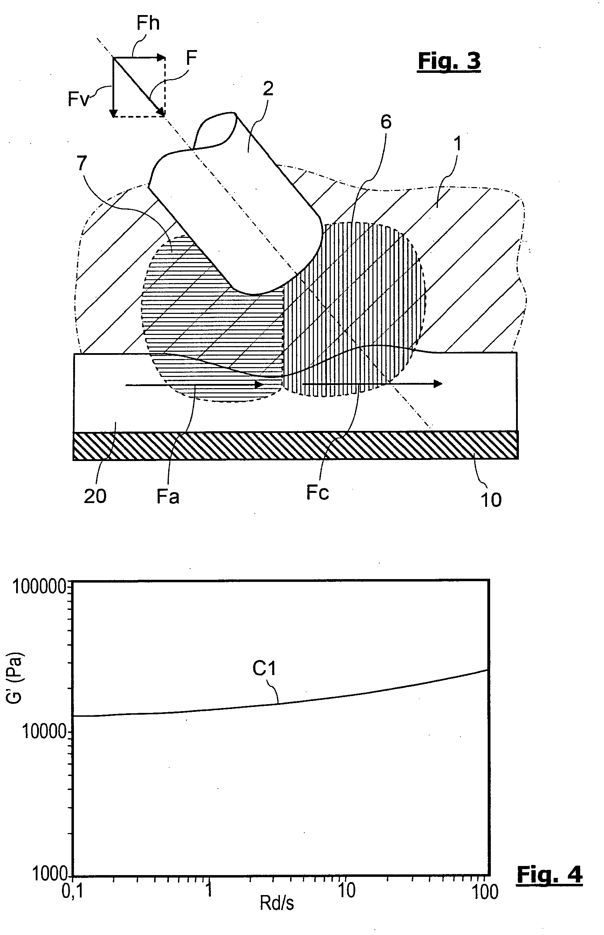

La figure 3 représente une zone de peau et de tissus au voisinage d'un os protégée par une couche de protection 20 selon un mode de réalisation. La couche de protection 20 comprend un gel de silicone obtenu par polymérisation au moins partielle de l'un ou l'autre de deux mélanges dont la composition est décrite dans le tableau 1 suivant :

**Tableau 1**

| Composant | Mélange 1 | Mélange 2 |
|---|---|---|
| polydiméthylsiloxane à terminaisons diméthyl-vinyles de viscosité supérieure à 20000 mPa.s | environ 15% | Environ 15% |
| polydiméthylsiloxane à terminaisons diméthyl-vinyles de viscosité entre 200 et 20000 mPa.s | environ 25% | Environ 25% |
| polydiméthylsiloxane à terminaisons triméthyles de viscosité inférieure à 100 mPa.s | environ 45% | Environ 25% |
| polydiméthylsiloxane à terminaisons triméthyles de viscosité supérieure à 20000 mPa.s | 0 | Environ 20% |
| silice de pyrogénation traitée triméthylsiloxy | environ 12% | Environ 12% |
| co-polydiméthylsiloxane-polyméthyl-hydrogéno-siloxane à terminaisons diméthyle-hydrogéno | environ 3% | Environ 3% |

La polymérisation partielle des mélanges 1 et 2 est obtenue à l'aide d'un système catalytique à base de platine complexé vinylesiloxanes.

La figure 4 représente une courbe C1 de variation de la rigidité ou composante élastique G' de la couche 20 à 35°C en fonction d'une fréquence de sollicitation. Sur la courbe C1, la rigidité de la couche 20 augmente d'environ 11000 à 20000 Pa lorsque la fréquence de sollicitation passe de 0 à 100 rd/s.

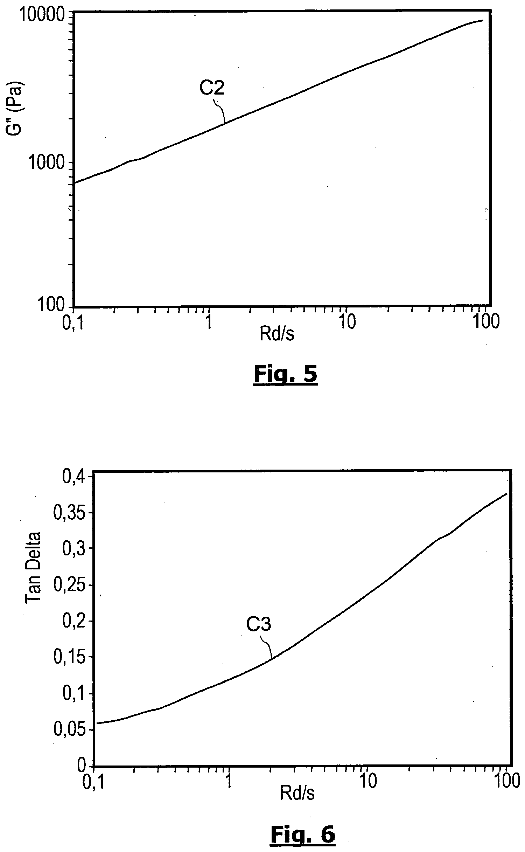

La figure 5 représente une courbe C2 de variation de la viscosité ou composante amortissante G" de la couche 20 à 35°C en fonction de la fréquence de sollicitation. Sur la courbe C2, la viscosité de la couche 20 augmente d'environ 700 à 8000 Pa lorsque la fréquence de sollicitation passe de 0 à 100 rd/s.

La figure 6 représente une courbe C3 de variation du facteur d'amortissement (rapport entre énergie dissipée et énergie restituée) ou Tan delta de la couche 20 à 35°C en fonction de la fréquence de sollicitation. Sur la courbe C3, le tan delta de la couche 20 augmente d'environ 0,06 à 0,38 lorsque la fréquence de sollicitation passe de 0 à 100 rd/s.

Les propriétés de la couche de protection 20 sont résumées dans le tableau 2 suivant :

**Tableau 2**

| | |
|---|---|
| Rigidité ou composante élastique à 35°C pour une fréquence de sollicitation comprise entre 0 et 100 rd/s | Environ 11000 à 20000 Pa |
| Viscosité ou composante amortissante à 35°C -pour une fréquence de sollicitation comprise entre 0 et 100 rd/s | Environ 700 à 8000 Pa |
| Facteur d'amortissement à 35°C pour une fréquence de sollicitation comprise entre 0 et 100 rd/s | environ 0, 06 à 0,38 |
| Adhésivité (tack) (g/cm2) | environ 100 à 115 |

La couche de protection 20 permet d'obtenir un bon compromis entre un matériau mou pour assurer un amortissement maximum, et à l'inverse, un matériau dur pour assurer une bonne résistance aux efforts de cisaillement et une bonne répartition des forces d'appui. A cet effet, l'épaisseur de la couche 20 peut être comprise entre 1 et 4 mm.

La surface de la couche de protection mise en contact avec la peau peut ne pas être lisse pour une meilleure adhésivité avec la peau, avec des aspérités comprises entre 2 et 20 µm de diamètre. Un tel état de surface peut être obtenu par une polymérisation libre de toute pression contre une surface lisse.

Il peut être constaté, comme représenté sur la figure 3, qu'une partie des forces de compression est absorbée par la couche de protection 20. En effet, si l'on compare les figures 2 et 3, la couche de protection 20 est déformée et l'épaisseur des tissus sous l'os est plus importante sur la figure 3. Il en résulte que les forces de compression 6 sont en partie absorbées par la couche de protection 20. Par ailleurs, une partie des efforts de cisaillement 7 est également absorbée par la couche 20. Il en résulte une diminution de la sollicitation dynamique répétée de la peau et des tissus plus en profondeur, qui induit une fatigue et peut entrainer des lésions. Il en résulte également une préservation des vaisseaux et donc le maintien de l'irrigation des tissus. L'adhésivité significative de la couche 20 permet de limiter d'une manière importante les frottements entre la peau et son environnement.

La couche de protection 20 peut être associée à un moyen permettant d'assurer son maintien sur la zone de la peau à protéger. Ce moyen peut être une pièce de tissu ou un autre gel de silicone plus adhésif fixé le long des bords de la couche 20.

L'épiderme est doté d'un grand nombre de récepteurs mécanosensibles (mécanorécepteurs) spécialisés pour trois types de sensations : la pression, la vibration et la sensibilité fine dite "tact", qui sont majoritairement orientés vers une finalité de protection. Il existe un réflexe mettant en jeu des mécanorécepteurs de l'épiderme, sensibles à la pression, le système nerveux et la vasodilatation des capillaires sanguins cutanés. Il s'avère que l'apparition de lésions cutanées et en particulier d'escarres résulte d'un dérèglement de ce réflexe. En effet, en l'absence de vasodilatation des capillaires sanguins cutanés, compensant une pression est exercée sur la peau, les capillaires se trouvent écrasés. Les tissus cutanés se trouvent alors insuffisamment irrigués, ce qui peut provoquer l'apparition d'une plaie dite "de dedans en dehors" correspondant à une escarre ou un ulcère de pression.

Il s'avère que la couche de protection 20 précédemment décrite, lorsqu'elle est appliquée directement sur la peau en un emplacement subissant une pression locale, permet de restaurer le réflexe de vasodilatation des capillaires sanguins cutanés, induit par l'application d'une pression locale, voire même d'améliorer cette vasodilatation, notamment chez les personnes atteintes de diabète. Il en résulte une bonne circulation sanguine cutanée, même en présence d'une pression, prévenant la formation d'escarre.

La couche de protection limite également les risques d'inhibition de ce réflexe de vasodilatation par la douleur. Elle améliore la répartition de charge et participe à l'absorption des efforts de cisaillement. Elle permet une restauration de la qualité de la peau la rendant moins sensible aux effets délétères des frottements et ou des appuis, grâce à sa grande capacité à hydrater la peau.

Il apparaîtra clairement à l'homme de l'art que la présente invention est susceptible de diverses variantes de réalisation et diverses applications. A ce sujet, il convient de noter que la couche de protection peut présenter une adhésivité inférieure à celle mentionnée dans le tableau 2, le maintien sur la peau de la couche de protection pouvant être assurée par d'autres moyens. La couche de protection peut également présenter une adhésivité supérieure, tout en évitant qu'elle soit excessive pour éviter le risque de formation de lésions lorsque la couche de protection est retirée de la peau.

## Revendications

1. Procédé de fabrication d'une couche de protection (20) contre les escarres,
**caractérisé en ce qu'**il comprend des étapes de formation d'un mélange, de polymérisation au moins partielle du mélange pour obtenir un gel polymère, et de formation d'une couche de protection (20) à partir du gel polymère, le mélange comprenant :
15% de polydiméthylsiloxane à terminaisons diméthyl-vinyles ayant une viscosité supérieure à 20000 mPa.s,
25% de polydiméthylsiloxane à terminaisons diméthyl-vinyles ayant une viscosité comprise entre 200 et 20000 mPa.s,
45% de polydiméthylsiloxane à terminaisons triméthyles,
12% de silice de pyrogénation traitée triméthylsiloxy, et
3% de co-polydiméthylsiloxane-polyméthyl-hydrogéno-siloxane à terminaisons diméthyle-hydrogéno,
le gel polymère présentant à 35°C lorsqu'une fréquence de sollicitation varie entre 0 et 100 rd/s, une rigidité ou composante élastique variant de 11000 à 20000 Pa, une viscosité ou composante amortissante variant de 700 à 8000 Pa, et un facteur d'amortissement ou Tan Delta variant de 0,06 à 0,38.

2. Procédé selon la revendication 1, dans lequel la couche de protection (20) présente une épaisseur comprise entre 1 et 4 mm.

3. Procédé selon l'une des revendications 1 et 2, dans lequel la couche de protection comprend une face présentant un état de surface obtenu par une polymérisation libre de toute pression contre une surface lisse.

4. Procédé selon l'une des revendications 1 à 3, dans lequel les 45% de polydiméthylsiloxane à terminaisons triméthyles présentent une viscosité inférieure à 100 mPa.s.

5. Procédé selon l'une des revendications 1 à 3, dans lequel les 45% de polydiméthylsiloxane à terminaisons triméthyles comportent environ 5/9ième de polydiméthylsiloxane à terminaisons triméthyles ayant une viscosité inférieure à 100 mPa.s, et environ 4/9ième de polydiméthylsiloxane à terminaisons triméthyles ayant une viscosité supérieure à 20000 mPa.s.

6. Procédé selon l'une des revendications 4 et 5, dans lequel la polymérisation est réalisée en présence de platine complexé vinylesiloxanes.

7. Couche de protection contre les escarres, **caractérisée en ce qu'**elle est obtenue par polymérisation au moins partielle d'un mélange comprenant :
15% de polydiméthylsiloxane à terminaisons diméthyl-vinyles ayant une viscosité supérieure à 20000 mPa.s,
25% de polydiméthylsiloxane à terminaisons diméthyl-vinyles ayant une viscosité comprise entre 200 et 20000 mPa.s,
45% de polydiméthylsiloxane à terminaisons triméthyles,
12% de silice de pyrogénation traitée triméthylsiloxy, et
3% de co-polydiméthylsiloxane-polyméthyl-hydrogéno-siloxane à terminaisons diméthyle-hydrogéno, le gel polymère présentant à 35°C, lorsqu'une fréquence de sollicitation varie entre 0 et 100 rd/s, une rigidité ou composante élastique variant de 11000 à 20000 Pa, une viscosité ou composante amortissante variant de 700 à 8000 Pa et un facteur d'amortissement ou Tan Delta variant de 0,06 à 0,38.

8. Couche de protection selon la revendication 7, présentant une épaisseur comprise entre 2 et 3 mm.

9. Couche de protection selon l'une des revendications 7 et 8, comprenant une face présentant un état de surface obtenu par une polymérisation libre de toute pression contre une surface lisse.

10. Couche de protection selon l'une des revendications 7 à 9, dans laquelle les 45% de polydiméthylsiloxane à terminaisons triméthyles présentent une viscosité inférieure à 100 mPa.s.

11. Couche de protection selon l'une des revendications 7 à 9, dans laquelle les 45% de polydiméthylsiloxane à terminaisons triméthyles comprennent :
environ 5/9ième de polydiméthylsiloxane à terminaisons triméthyles ayant une viscosité inférieure à 100 mPa.s, et
environ 4/9ième de polydiméthylsiloxane à terminaisons triméthyles ayant une viscosité supérieure à 20000 mPa.s.

12. Couche de protection selon l'une des revendications 10 et 11, dans lequel la polymérisation est réalisée en présence de platine complexé vinylesiloxanes.

13. Produit pour restaurer ou renforcer le réflexe de vasodilatation des capillaires sanguins cutanés, induit par l'application d'une pression locale,
**caractérisé en ce qu'**il comprend une couche de protection (20) selon l'une des revendications 7 à 12.

## Patentansprüche

1. Verfahren zur Herstellung einer Schutzschicht (20) gegen Dekubitus,
**dadurch gekennzeichnet, dass** es die Schritte des Bildens eines Gemisches, des zumindest teilweisen Polymerisierens der Mischung zum Erhalten eines Polymergels und des Bildens einer Schutzschicht (20) aus dem Polymergel umfasst, wobei die Mischung Folgendes umfasst:
15 % Polydimethylsiloxan mit endständigen Dimethylvinylgruppen mit einer Viskosität von mehr als 20000 mPa·s,
25 % Polydimethylsiloxan mit endständigen Dimethylvinylgruppen mit einer Viskosität zwischen 200 und 20000 mPa·s,
45 % Polydimethylsiloxan mit endständigen Trimethylgruppen,
12 % Trimethylsiloxy-behandelte pyrogene Kieselsäure und
3 % Co-Polydimethylsiloxan-Polymethyl-Wasserstoffsiloxan mit endständigen Dimethyl-Wasserstoffgruppen,
wobei das Polymergel bei 35 °C, wenn eine Beanspruchungsfrequenz zwischen 0 und 100 rd/s variiert, eine Steifigkeit oder elastische Komponente variierend zwischen 11000 und 20000 Pa, eine Viskosität oder Dämpfungskomponente variierend zwischen 700 und 8000 Pa und einen Dämpfungsfaktor oder Tan Delta variierend zwischen 0,06 und 0,38 aufweist.

2. Verfahren nach Anspruch 1, bei dem die Schutzschicht (20) eine Dicke zwischen 1 und 4 mm aufweist.

3. Verfahren nach einem der Ansprüche 1 und 2, wobei die Schutzschicht (20) eine Fläche mit einer Oberflächenbeschaffenheit umfasst, die durch Polymerisation ohne jeglichen Druck gegen eine glatte Oberfläche erhalten wird.

4. Verfahren nach einem der Ansprüche 1 bis 3, wobei die 45 % des Polydimethylsiloxans mit endständigen Trimethylgruppen eine Viskosität von weniger als 100 mPa·s aufweisen.

5. Verfahren nach einem der Ansprüche 1 bis 3, wobei die 45 % des Polydimethylsiloxans mit endständigen Trimethylgruppen etwa 5/9 Polydimethylsiloxan mit endständigen Trimethylgruppen mit einer Viskosität von weniger als 100 mPa·s und etwa 4/9 Polydimethylsiloxan mit endständigen Trimethylgruppen mit einer Viskosität von mehr als 20000 mPa·s umfassen.

6. Verfahren nach einem der Ansprüche 4 und 5, wobei die Polymerisation in Gegenwart von Platin-komplexierten Vinylsiloxanen durchgeführt wird.

7. Schutzschicht gegen Dekubitus,
**dadurch gekennzeichnet, dass** sie durch zumindest partielle Polymerisation eines Gemischs erhalten wird, umfassend:
15 % Polydimethylsiloxan mit endständigen Dimethylvinylgruppen mit einer Viskosität von mehr als 20000 mPa·s,
25 % Polydimethylsiloxan mit endständigen Dimethylvinylgruppen mit einer Viskosität zwischen 200 und 20000 mPa·s,
45 % Polydimethylsiloxan mit endständigen Trimethylgruppen,
12 % Trimethylsiloxy-behandelte pyrogene Kieselsäure und
3 % Co-Polydimethylsiloxan-Polymethyl-Wasserstoffsiloxan mit endständigen Dimethyl-Wasserstoffgruppen,
wobei das Polymergel bei 35 °C, wenn eine Beanspruchungsfrequenz zwischen 0 und 100 rd/s variiert, eine Steifigkeit oder elastische Komponente variierend zwischen 11000 und 20000 Pa, eine Viskosität oder Dämpfungskomponente variierend zwischen 700 und 8000 Pa und einen Dämpfungsfaktor oder Tan Delta variierend zwischen 0,06 und 0,38 aufweist.

8. Schutzschicht nach Anspruch 7, die eine Dicke zwischen 2 und 3 mm aufweist.

9. Schutzschicht nach einem der Ansprüche 7 und 8, umfassend eine Fläche mit einer Oberflächenbeschaffenheit, die durch Polymerisation ohne jeglichen Druck gegen eine glatte Oberfläche erhalten wurde.

10. Schutzschicht nach einem der Ansprüche 7 bis 9, wobei die 45 % des Polydimethylsiloxans mit endständigen Trimethylgruppen eine Viskosität von weniger als 100 mPa·s aufweisen.

11. Schutzschicht nach einem der Ansprüche 7 bis 9, wobei die 45 % des Polydimethylsiloxans mit endständigen Trimethylgruppen Folgendes umfassen:
etwa 5/9 Polydimethylsiloxan mit endständigen Trimethylgruppen mit einer Viskosität von weniger als 100 mPa·s, und
etwa 4/9 Polydimethylsiloxan mit endständigen Trimethylgruppen mit einer Viskosität von mehr als 20000 mPa·s.

12. Schutzschicht nach einem der Ansprüche 10 und 11, wobei die Polymerisation in Gegenwart von Platin-komplexierten Vinylsiloxanen durchgeführt wird.

13. Produkt zur Wiederherstellung oder Stärkung des Vasodilatationsreflexes der kutanen Blutkapillaren, ausgelöst durch die Anwendung von lokalem Druck,
**dadurch gekennzeichnet, dass** es eine Schutzschicht (20) nach einem der Ansprüche 7 bis 12 umfasst.

## Claims

1. A method for manufacturing a protective layer (20) against bedsores,
**characterized in that** it comprises steps of forming a mixture, of polymerizing at least partially the mixture to obtain a polymer gel, and of forming a protective layer (20) using the polymer gel, the mixture comprising:
15% of dimethyl-vinyl-terminated polydimethylsiloxane with a viscosity greater than 20,000 mPa.s,
25% of dimethyl-vinyl-terminated polydimethylsiloxane with a viscosity between 200 and 20,000 mPa.s,
45% of trimethyl-terminated polydimethylsiloxane,
12% of trimethylsiloxy-treated pyrogenic silica, and
3% of dimethyl-hydrogen-terminated co-polydimethylsiloxane-polymethyl-hydrogen-siloxane,
the polymer gel having at 35°C, when a stress frequency varies between 0 and 100 rd/s, a rigidity or elastic component varying from 11,000 to 20,000 Pa, a viscosity or cushioning component varying from 700 to 8,000 Pa, and a cushioning factor or Tan Delta varying from 0.06 to 0.38.

2. Method according to claim 1, wherein the protective layer (20) has a thickness between 1 and 4mm.

3. Method according to one of claims 1 and 2, wherein the protective layer comprises a face having a surface state obtained by polymerization free from any pressure against a smooth surface.

4. Method according to one of claims 1 to 3, wherein the 45% of trimethyl-terminated polydimethylsiloxane have a viscosity lower than 100 mPa.s.

5. Method according to one of claims 1 to 3, wherein the 45% of trimethyl-terminated polydimethylsiloxane have about 5/9^{ths} of trimethyl-terminated polydimethylsiloxane with a viscosity lower than 100 mPa.s, and about 4/9^{ths} of trimethyl-terminated polydimethylsiloxane with a viscosity greater than 20,000 mPa.s.

6. Method according to one of claims 4 and 5, wherein the polymerization is performed in the presence of platinum vinylsiloxane complex.

7. A protective layer against bedsores, **characterized in that** it is obtained by polymerizing at least partially a mixture comprising:
15% of dimethyl-vinyl-terminated polydimethylsiloxane with a viscosity greater than 20,000 mPa.s,
25% of dimethyl-vinyl-terminated polydimethylsiloxane with a viscosity between 200 and 20,000 mPa.s,
45% of trimethyl-terminated polydimethylsiloxane,
12% of trimethylsiloxy-treated pyrogenic silica, and
3% of dimethyl-hydrogen terminated co-polydimethylsiloxane-polymethyl-hydrogen-siloxane, the polymer gel having at 35°C, when a stress frequency varies between 0 and 100 rd/s, a rigidity or elastic component varying from 11,000 to 20,000 Pa, a viscosity or cushioning component varying from 700 to 8,000 Pa and a cushioning factor or Tan Delta varying from 0.06 to 0.38.

8. Protective layer according to claim 7, having a thickness between 2 and 3mm.

9. Protective layer according to one of claims 7 and 8, comprising a face having a surface state obtained by polymerization free from any pressure against a smooth surface.

10. Protective layer according to one of claims 7 to 9, in which the 45% of trimethyl-terminated polydimethylsiloxane have a viscosity lower than 100 mPa.s.

11. Protective layer according to one of claims 7 to 9, in which the 45% of trimethyl-terminated polydimethylsiloxane comprise:
about 5/9^{ths} of trimethyl-terminated polydimethylsiloxane with a viscosity lower than 100 mPa.s, and
about 4/9^{ths} of trimethyl-terminated polydimethylsiloxane with a viscosity greater than 20,000 mPa.s.

12. Protective layer according to one of claims 10 and 11, wherein the polymerization is performed in the presence of platinum vinylsiloxane complex.

13. A product for restoring or strengthening the vasodilatation reflex of skin blood capillaries, induced by applying local pressure,
**characterized in that** it comprises a protective layer (20) according to one of claims 7 to 12.
